# EUROPEAN PATENT SPECIFICATION

(11) **EP 4 150 348 B1**
(45) Date of publication and mention of the grant of the patent: **12.11.2025**
(21) Application number: 21724328.6
(22) Date of filing: 12.05.2021
(51) Int. Cl.: G01N 33/68

(54) **SAMPLE PREPARATION FOR MASS SPECTROMETRY**
PROBENVORBEREITUNG FÜR MASSENSPEKTROMETRIE
PRÉPARATION D'ÉCHANTILLONS POUR SPECTROMÉTRIE DE MASSE

(30) Priority: 13.05.2020 EP 20174484
(43) Date of publication of application: 22.03.2023
(73) Proprietor: PreOmics GmbH, 82152 Planegg/Martinsried (DE)
(72) Inventor: KULAK, Nils A., 80686 München (DE); HARTINGER, Katrin, 81249 München (DE); KÄSEMANN, Martin, 40789 Monheim (DE); JOHANSSON, Sebastian, 82284 Grafrath (DE); JOHANSSON, Jasmin, 82284 Grafrath (DE)
(74) Representative: Dentons Patent Solutions Rechtsanwaltsgesellschaft mbH
(86) International application number: PCT/EP2021/062677
(87) International publication number: WO 2021/228969

(56) References cited:
- WO-A1-2020/002577
- WO-A2-2004/070352
- WO-A2-2008/156688
- WO-A2-2015/091876
- US-A1- 2014 017 716
- US-B2- 6 884 357
- GONG CHENG ET AL: "Construction of a high-performance magnetic enzyme nanosystem for rapid tryptic digestion", SCIENTIFIC REPORTS, vol. 4, no. 1, 6 November 2014 (2014-11-06), XP055726257, DOI: 10.1038/srep06947
- ZAKREVSKII V A ET AL: "On the possible effect of a magnetic field on the breaking of mechanically loaded covalent chemical bonds", PHYSICS OF THE SOLID STATE, NAUKA/INTERPERIODICA, MO, vol. 44, no. 11, 1 November 2002 (2002-11-01), pages 2083 - 2086, XP019310540, ISSN: 1090-6460
- YONGLE PANG ET AL: "Pepsin-Containing Membranes for Controlled Monoclonal Antibody Digestion Prior to Mass Spectrometry Analysis", ANALYTICAL CHEMISTRY, vol. 87, no. 21, 22 October 2015 (2015-10-22), pages 10942 - 10949, XP055726215, ISSN: 0003-2700, DOI: 10.1021/acs.analchem.5b02739

## Description

The present disclosure relates to a method of preparing a sample for an analytic procedure, said sample comprising at least one protein, polypeptide or peptide molecule, and said method comprising fragmenting said molecule using at least one moving magnetic body.

Within the last years, bioanalytical procedures developed at a fast pace. In particular, mass spectrometry (MS)-based proteomics emerged as a powerful analytical tool for various pharmaceutical and clinical applications. While traditional approaches including immuno- and enzyme-based assays are limited to the analysis of single proteins at a time that have already been specified in advance, untargeted mass spectrometry opens up completely new perspectives by detecting hundreds to thousands of proteins within a single measurement in a completely unbiased manner. Novel MS strategies are, inter alia, increasingly applied for the analysis of biomarkers in body fluids such as urine, plasma or serum in order to assess and monitor an individual's health state. A second broad field of application is tracking host cell protein (HCP) impurities in therapeutic proteins throughout the entire production and purification process to guarantee safety and quality of drug products.

Besides complex samples, MS-based proteomics is also used for single protein analysis, especially for therapeutic proteins where extensive and comprehensive characterization of the drug substance is indispensable for patient safety. Since this approach focuses on the detailed sequence and composition of a single protein including post-translational modifications (PTMs), sample preparation needs to be optimized regarding these specific requirements e.g. by boosting preparation efficiency for maximum sequence coverage or by reducing the introduction of artificial modifications.

Enzymatic protein digestion presents a crucial step in most sample preparation workflows for MS-based proteomics whereby specific endoproteinases (often referred to as proteases) are employed to hydrolyse proteins on their protein backbone to yield polypeptides or peptides. Most proteases used in proteomics are sequence-specific endoproteinases and serve to prevent an increase of the complexity of the sample beyond the level of MS capabilities. For bottom-up proteomic strategies, trypsin of mammalian origin (porcine or bovine) is the most widespread protease. Trypsin cleaves specifically at the carboxylic side (C-terminus) of arginine (R) and lysine (K) residues and thus generates positively charged peptides with an average length of approximately nine amino acids. Other proteases including LysC, GluC, AspN, ArgC and chymotrypsin are also used in MS sample preparation, however, to a lesser extent and often in order to complement tryptic sample information. Manufacturers aim to improve protease purity while retaining enzyme activity using recombinant protein expression systems instead of natural protein from mammalian or bacterial sources, but are often confronted by the challenge of expressing highly active proteolytic enzymes.

Complete coverage MS-based proteomics largely depends on efficient proteolysis and the resulting sequence coverage during data analysis. Most sample preparation protocols therefore rely on high protease content and lengthy digestion times of up to 24 hours. Proteolytic digestion thus represents a critical step with a significant influence on variability of proteomic sample preparation seen across laboratories and enterprises.

Several further parameters are known to influence quality and reproducibility of enzymatic proteolysis. Among others, buffer composition is a critical aspect since proteases are highly sensitive towards pH and salt conditions as well as to the presence of detergents and chaotropic reagents. Furthermore, while digestion efficiency can be heavily increased by higher enzyme concentrations, additional protein contamination from the enzyme source (e.g. porcine proteins) is introduced at the same time, thus presenting challenges and limitations of the art-established digestion strategies. Also the protease as such can lead to undesirable signals in the mass spectrum which e.g. overlay signals from the sample
In contrast to classical bottom-up proteomics, top-down proteomic approaches are used to analyse intact proteins in the MS instrument. This application is highly attractive because it allows investigation of complete proteins and to determine modification patterns. However, properties of the protein to be analysed strongly influence the quality of analysis, and examining a complex protein sample remains challenging. Intact proteins do not behave uniformly under MS-compatible liquid chromatography (LC) conditions and are difficult to separate which leads to highly complex samples and a very broad dynamic range at any given moment of data acquisition . Furthermore, intact proteins are only inefficiently ionized by electrospray ionization (ESI) which is the method of choice as a soft ionization technique when the MS is coupled to LC setups. Finally, intact proteins lead to highly complex isotope patterns at the MS analysis side making it very difficult to interpret mass spectra. As a consequence, top-down proteomics of complex protein samples is not applicable in a wide range of settings and is limited to expert laboratories.

Middle-down approaches represent a highly attractive compromise between top-down and bottom-up proteomics, promising high sequence coverage in conjunction with well-established analytical systems. Middle-down approaches involve proteolytic digestion like bottom-up approaches, but in a more restricted manner and aim for larger peptide fragments. Here, structure-specific enzymes are often chosen to selectively cleave at one or few sites of a protein, such as papain or the IdS protease for antibody analysis. A major challenge, however, is the identification of generically suitable proteases for a broad range of proteins to be analysed. This challenge has been addressed to a certain extent by chemical means of protein hydrolysis.

Classical chemical means to hydrolyse proteins involve strong acids at elevated temperatures. This leads to nearly random cleavage of the protein backbone. Typically, proteins are boiled in strong acids such as highly concentrated muriatic acid (10 M HCl) for approximately 1-24 h depending on the level of degradation to be achieved.

This process can be partially regulated by acid concentration, temperature and time, though hydrolysis occurs randomly leading to extremely complex samples of hydrolysed proteins. This process can be performed in a more rapid yet less streamlined manner using microwave-assisted protein hydrolysis. Here, microwaves are used to increase the rate of hydrolysis due to water excitation. The major challenge hereby remains the complexity of cleavage possibilities resulting from random hydrolysis. This typically exceeds the capacity of even the most advanced MS instruments.

Most LC-MS-instruments used for bottom-up proteomics are optimized for the identification of tryptic peptides, i.e., peptides obtained by digestion of a protein with trypsin. Peptide sequences of approximately 6 to 40 amino acid (AA) sequence length giving rise to doubly charged peptide ions (one positive charge at the K/R-residue, one positive charge at N-terminus) can be separated very efficiently using reversed-phase (C18) chromatography, coupled via ESI to a high-resolution mass spectrometer. Proteases leading to shorter peptides (<6 AA) have the disadvantage that peptides are typically not any more protein-specific but instead occur in many potential proteins. Proteases leading to longer peptides (>100 AA) may cause irreversible binding of peptides to reversed-phase chromatography columns and consequently, inefficient ESI.

The aim of middle-down proteomics would be to generate uniform and reproducible peptide lengths of approximately 30 to 100 AA lengths across all proteins present in the sample. This in turn would lead to peptide samples which are well suited for reversed-phase chromatography, stable ESI and MS-analysis while significantly reducing sample complexity and increasing sequence coverage.

Gong et al., Scientific Reports 4, 2014, doi:10.1038/ srep06947 describes the construction of a high-performance magnetic enzyme nanosystem for rapid tryptic digestion.

WO2008156688 A2 uses magnetic field to move the microparticles to separate biological analytes.

WO2004070352 A2 concerns sample preparation for MS, using separation processes.

US2014017716 A1 concerns sample preparation for MS, using separation processes involving magnetic beads. These documents do not involve fragmentation related collisions with magnetic bodies.

In view of the shortcomings of the known approaches of sample preparation for MS, the technical problem underlying the present invention can be seen in the provision of improved means and methods of preparing a sample for analysis in a mass spectrometer, such methods including fragmenting of chain molecules and macromolecules, including those of biological origin.

As can be seen from the Examples included in this specification, this technical problem is solved by the subject-matter of the claims, which defines the invention, presented further below.

More specifically, in a first aspect, the present invention provides a method of preparing a sample for an analytic procedure, said sample comprising at least one protein, polypeptide or peptide molecule, and said method comprising (a) fragmenting said molecule using at least one moving magnetic body, wherein said at least one magnetic body performs a fluctuating or oscillating motion, wherein said motion is triggered by a fluctuating or oscillating magnetic field, wherein said magnetic body collides with said molecule, and wherein under such circumstances energy is transferred to said molecule triggering or contributing to fragmenting said molecule.

The term "preparing" generically refers to pre-processing of a sample such that the result thereof can be analysed. Typically, proteins and polypeptides as well as longer peptides are molecules the size of which is not optimal for analytical purposes such as analysis in a mass spectrometer. At least for that reason, samples generally require a step of preparation prior to them being fed into a mass spectrometer. Such preparation includes reducing the size of the analyte molecules which is done by fragmenting. Conventionally, fragmenting is done by adding a protease such as trypsin. A protease is an enzymatic means of fragmenting, shortcomings of which are reviewed in the introductory section above.

Deviant from such art-established pre-processing, preparing in accordance with the present invention employs at least one moving magnetic body. In terms of implementation, this may be a single magnet in the simplest case. This option as well as alternatives thereto are described in more detail further below.

The present inventors surprisingly found that a moving magnetic body is capable of triggering fragmentation, i.e., the breaking of one or more covalent bonds in said molecule. This has distinct advantages, one of them being that the abovementioned protease becomes dispensable. Indeed, in the simplest implementation of the invention, no chemical or biological agents at all are used for the purpose of fragmenting proteins, polypeptides or longer peptides. Indeed, and as reviewed herein above, the art relies on biological agents, more specifically proteases such as trypsin, for cleaving proteins. Also the use of chemicals such as CNBr has been described for that purpose. Cleavage or fragmentation in accordance with the invention on the other hand may be implemented without enzymatic catalysis and without chemical agents which trigger cleavage of proteins, polypeptides or peptides.

It is understood that by the terms "chemical agents" and "chemical" reference is made to compounds such as CNBr (further compounds capable of cleaving proteins, polypeptides or peptides are disclosed further below) which trigger cleavage under conditions (e.g. ambient temperature, atmospheric pressure, presence of water, buffers, salts) where otherwise cleavage would not or not significantly occur. In other words, such chemical would deliberately be added for the purpose of cleaving proteins, polypeptides or peptides.

Fragmenting may directly lead to stable fragments or to intermediate reactive species which in turn react with other molecules present to yield stable products. Other molecules may include water or more generally speaking, any molecules which are capable of forming stable adducts with reactive species to the extent they are formed.

The term "peptide" refers to polycondensates of up to 30 amino acids, whereas polypeptides comprise more than 30 amino acid building blocks. As such, "longer peptides" refers to peptides consisting of 15 to 30, 20 to 30 or 25 to 30 amino acids. The term "protein" embraces polypeptides but extends to higher order structures, e.g. non-covalent associations or oligomers of a plurality of identical and/or different proteins.

Modifications may be present on any one of peptides, polypeptides and protein and include, but are not confined to post-translational modifications as they occur in biological systems. Examples include glycosylation, phosphorylation and modifications with lipophilic moieties such as prenylation. Upon fragmentation, the mentioned higher-order structures as it may be present in proteins (oligomers) is generally, but not necessarily lost. Local structure, such as secondary structure might be retained, as may be the capability of certain domains or motifs to interact with cognate binding partners.

In terms of building blocks, preference is given to the 20 proteinogenic amino acids. Yet, amino acids, generally α-amino acids, not belonging to this set of 20 amino acids occur naturally and may be present as well. Examples include ornithine, citrulline, hydroxyproline, selenocysteine, oxidized methionine, and deaminated asparagine.

This repertoire may be further expanded, e.g. by the mentioned post-translational modifications. These include for example the phosphorylated forms of serine, threonine and tyrosine, glycosylated amino acids, methylated lysine, and methylated arginine.

In terms of links between amino acids, this is generally the canonic main chain peptide bond between the α-carboxylate of one amino acid and the α-amino group of the subsequent amino acid (direction from N- to C-terminus). Less common, but envisaged links include iso-peptide bonds, i.e., peptide bonds which involve side-chain amino groups and/or side-chain carboxylates.

Fragmentation by the method of this invention may occur at main chain peptide bonds, but other bonds may fragment as well.

Indeed, the covalent bond that is cleaved is not particularly limited. Included are single bonds as well as double and triple bonds, which all may between atoms of the same type (such as a C-C bond) or different atoms (such as a C-N bond or a C-O bond). Bonds occurring in functional groups linking building blocks - in addition to peptide bonds - are ester bonds including phospho-esters, and glycosidic bonds. Phospho-ester bonds occur e.g. in phosphorylated Ser, Thr and Tyr residues, and glycosidic bonds in glycosylated amino acids. It is understood that bonds occurring in such functional groups generally have a lower binding energy as compared to, e.g. a C=C bond, and are therefore amenable to cleavage with the method of the invention when operated in a manner which transfers less energy to the reaction mixture.

Energies contained in covalent bonds are known; these energies also define the energy required for cleavage, especially in the absence of a catalyst. Most covalent bond energies fall into the interval from 100 to 1200 kJ/mol; see, e.g., Chemistry: Atoms First 2e, ISBN 978-1-947172-63-0. To give a few examples of covalent bond energies, a C-C bond has a bond energy of 345 kJ/mol, a C-N bond of 290 kJ/mol, and a C-O bond of 350 kJ/mol.

In a preferred embodiment, said analytic procedure is a spectrometric and/or spectroscopic method. A preferred spectrometric method is mass spectrometry (MS). Preferred spectroscopic methods include NMR spectroscopy and/or UV/vis spectroscopy. One or more of these methods may be used subsequently. Particularly preferred is MS.

For conventional mass spectrometric applications, a preferred size range of peptides is 5 to 30 amino acids. A preferred median size is about 12 amino acids. As such, it is apparent that also peptides, to the extent their size is above that range, are preferably fragmented prior to analysis.

Having said that, and as stated in the introductory part above, there are instances (also referred to as "middle-down approach") where longer molecules such as polypeptides of a length between 30 and 100 amino acids are to be fed into the mass spectrometer. In these cases, peptides are not required to be fragmented, but only polypeptides and proteins to the extent their size exceeds the size which is desirable for these particular implementations. It is particularly in such cases where the art-established means of protein fragmentation fail to provide a satisfactory solution. Yet, it is highly desirable to obtain fragments in this size range because they are unique (specific for a single protein) with greater likelihood than shorter fragments. In other words, less fragments are sufficient for good sequence coverage. Advantageously, the present invention fills this gap.

More generally speaking, the preferred size range is also dependent on the choice of the analytical method (usually liquid chromatography) preceding mass spectrometry. To the extend reverse phase material is used, C18 material is particularly apt for peptides, whereas C8 material can also be used for proteins. Orbitrap instruments have the best resolving power in a lower range of *m*/*z*, wherein TOF instruments do so at higher ranges. *"m*/*z"* designates the mass (*m*) to charge (*z*) ratio which is the property generally governing the separation of ions in a mass spectrometer.

In terms of *m*/*z,* a preferred range of fragment sizes is about 300 to about 1700 Thomson (Th). As regards the mentioned middle-down approach, and assuming peptide ions to be higher charged, a preferred range would be between about 600 and about 2000 Th.

As a consequence of the above, the desired number of fragments for a given starting molecule will depend on the one hand on the size of the starting molecule and on the other hand on the average fragment size which is desired under the circumstances given. For the sake of completeness, it is noted that the number of fragments obtained from a given molecule is not particularly limited and may vary between two fragments and any higher number such as 3, 4, 5, 6, 7, 8, 9, 10, 20, 50, 100, 200, 500, 1000 or more fragments.

Of note, and this is also shown in the Examples, a given protein does not always fragment in the same manner when applying the method of the present invention. A given molecule of a molecular species might break at positions which are partly or completely different from the positions where another molecule of the same molecular species breaks under the same conditions. Also, the number of fragments obtained may differ. This is a further advantage when comparing to the art-established proteolytic digestion which usually or with greater likelihood yields the same fragments for a given protein under given conditions. In particular, proteases generally exhibit a sequence-dependent specificity whereas the method of the present invention usually does not or to a lesser and/or different degree.

As shown in Example 2, the method of the invention delivers more than 1000 different fragments of a protein with about 250 amino acids - as compared to a low two-digit number in case of a conventional tryptic digest. The great variety of fragments facilitates *de novo* sequencing by mass spectroscopy which is a further aspect of this invention and disclosed further below.

Owing to the non-enzymatic and non-chemical cleavage process of the invention, fragmentation patterns may be generated which previously were not attainable. In this context, the term "fragmentation pattern" refers to both the sites of fragmentation as well as the average size and size distribution of the fragments obtained from a given molecule.

The term "at least one protein, polypeptide or peptide molecule" embraces applications which use exactly or approximately one molecule, also termed "single molecule applications". Where there is no need to study or handle individual molecules, but to fragment small numbers of molecules, amounts from 1 fmol to 1 µmol are embraced by the term "at least one molecule". Also embraced are macroscopic amounts, e.g. in the range from 1 µmol to 10⁶ mol per molecule species.

The molecules to be cleaved may belong to a single molecular species, may form a family of more or less closely related molecules, or may be entire proteomes. Indeed, the analysis of proteomes is of particular interest and a field for which mass spectrometry is particularly apt. Yet, as regards the process of sample preparation leading up to the actual mass spectrometric analysis, there is room for improvement. This is addressed by the novel method of fragmenting of this invention.

The term "mass spectrometer" has its art-established meaning and is not particularly limited. It refers to a device which allows to separate charged analytes on the basis of the ratio *m*/*z, m* being the mass and z the charge of said analyte (Unit: Thomson). As is apparent from the above, the analytes to be fed into the mass spectrometer are the peptides (and possibly polypeptides) obtained by fragmentation. A mass spectrometer generally comprises, in addition to a device performing the mentioned separation, a device for ionization of the analytes comprised in a sample. Yet further components may be means for storing or filtering ions.

In a preferred embodiment, the sample to be processed is liquid. Liquid handling is facilitated by containers. Accordingly, in a preferred embodiment, said at least one molecule and said at least one magnetic body are located in a reactor. The term "reactor" as used herein generically refers to an enclosure or container where said at least one molecule and said at least one magnetic body are located and allowed to interact ("react") in accordance with the invention. Otherwise, the term is not particularly limiting and embraces vessels, vessels with a closed bottom, vessels with a lid, vessels with a closed bottom and a lid, entirely closed or sealed vessels, tubular elements, and elements with at least two openings allowing for continuous liquid flow through an accordingly designed reactor. Reactors may also be implemented as microfluidic devices, i.e., miniaturized devices comprising one or more channels with openings, optionally with widenings or vessels and/or valves.

The present inventors that nucleic acids are similarly amenable to magnetic fragmenting. As such, the present invention, in a related aspect, provides a method of preparing a sample for an analytic procedure, said sample comprising at least one nucleic acid molecule, and said method comprising (a) fragmenting said molecule using at least one moving magnetic body.

The term "nucleic acid" has its common meaning. It includes DNA and RNA as well chimeric molecules. In terms of lengths, it comprises oligonucleotides and polynucleotides. Also embraced are RNA molecules with specific functions and/or specific structural features such as mRNA, tRNA, siRNA and miRNA.

In a preferred embodiment, said magnetic body is (a) a single magnet; or (b) an assembly of particles at least one of which is a magnet and assembling of said particles is mediated by magnetic fields of said at least one magnet. In brief, in a magnetic field, the obtained assembly essentially behaves like a single magnet; for details see further below. In terms of size, said particles will be larger than the molecules to be fragmented (and smaller than the vessel or reactor where the method is performed).

A magnet is a piece of ferri-, ferro- or paramagnetic material. Sizes of single magnets may vary widely and may be appropriately chosen in dependency of the dimension of the reactor or vessel to be used. As discussed in more detail below, when practicing the method of the invention, preferably a reactor or vessel is employed which holds the at least one molecule to be fragmented and the at least one magnetic body.

In terms of relative size, it is preferred that the largest dimension of the magnetic body fits through the smallest passage or cross-section in said reactor or vessel. Smaller than the smallest dimension preferably means 2/3, ½, 1/3, ¼, or 10% of said smallest dimension or cross-section of said reactor, vessel or tube. Such setup generally provides for free or substantially free motion. Preferably, free or substantially free motion occurs around or along at least two, at least three, at least four, at least five or preferably all six axes of translational and rotational motion, and wherein preferably said free or substantially free motion includes translation along at least two axes.

To explain, for a point-like object, there are three degrees of motional freedom, i.e., translation in three independent directions spanning the three-dimensional space. For an extended object, there are three further degrees of freedom which can be defined in terms of three independent axes of rotation.

To the extent present, other material in the reactor or reaction mixture may be taken into account when choosing the size of the magnetic body, such other material including further components of the sample in addition to the molecule(s) to be cleaved and/or non-magnetic particles (also referred to as non-magnetic beads; see further below).

For the sake of completeness, exemplary values of the size of a useful magnet are given here to be between 0.1 mm and 10 cm such as between 0.2 mm and 2 cm, including any of the following values and ranges defined thereby: 0.3 cm, 0.4 cm, 0.5 cm, 0.6 cm, 0.7 cm, 0.8 cm, 0.9 cm, and 1 cm. The same preferred sizes and size ranges apply to any magnetic body considered herein, also to implementations where use is made of a plurality of magnetic bodies and/or magnets. Generally, these lengths refer to the largest extension of said magnetic body.

Alternatively, a plurality of magnets such as those defined above may be used. Exemplary, but non-limiting numbers are in the one-digit and two-digit range such as 2, 3, 4, 5, 6, 7, 8, 9, and 10. Also more than 10 such as 20, 30, 40, or 50 magnets may be used. These numbers refer to one reactor or vessel, also in case arrangements of vessels or reactors (such as microtiter plates) are used.

It is understood that the number of magnetic bodies or magnets, to the extent it is specified, is limiting. In other words, while the method of the first aspect may comprise other measures, such possibility does not extend to the option of more magnetic bodies or magnets being present than expressly specified.

The magnets of such a plurality of magnets may be identical or different from each other.

The same applies to magnetic bodies in general, to the extent a plurality thereof is to be used. To give a specific example, a single magnet may be combined with a single ferro- or ferrimagnetic bead. This provides for more rigorous motion of the magnet while the parameters controlling the magnetic field are left unchanged.

In terms of shape of said magnet, there are no particular limitations, wherein preference is given to those shapes which do not negatively interfere with the free motion of the magnet. Exemplary shapes include sticks, bars, rods, rods with rounded ends, cubes, cuboids, prisms, spheres, elongate and oblate ellipsoids, disks, tetrahedrons, octahedrons, dodecahedrons, and icosahedrons.

Such setup has still to be distinguished from the above disclosed "assembly". The latter refers to a setup where a number, typically a large number (e.g. hundreds or thousands) of particles assemble under the influence of a single or a few (such as tens) magnets (possibly re-enforced by the magnetic field in accordance with the invention) into a single magnetic body which essentially behaves like a single magnet. For the sake of completeness, also a plurality of magnetic bodies, each of them or part of them resulting from an assembly as described above, may be employed. Yet, this is not very likely under many circumstances. In particular, and in case no measures are taken to prevent this from happening, placing more than one assembly into the same reactor or vessel may lead to the formation of a single bigger assembly (which in turn also behaves like a magnet once in a magnetic field).

In a further preferred embodiment, (a) said magnet comprises or consists of ferromagnetic material or ferrimagnetic material; (b) the particles of said assembly as defined above comprise or consist of a material selected from ferromagnetic materials, ferrimagnetic materials, superparamagnetic materials, paramagnetic materials and/or diamagnetic materials; and/or (c) said magnet and/or said particles are coated, preferably with a coating selected from (i) a coating conferring chemical stability; (ii) a coating conferring mechanical stability or hardness; (iii) a coating with a catalyst; (iv) a coating with a nucleic acid such as a probe and/or primer; (v) a coating with a chelating agent such as IMAC, TiO₂ and ZrO₂; (vi) a coating with a chromatographic material, preferably selected from (1) reversed phase groups such as C18, C8, Benzene; (2) HILIC groups such as hydroxyl groups; (3) cation ion-exchange groups such as sulfonic acid, phosphoric acid, carboxylic acid; (4) anion-exchange groups such as primary, secondary, tertiary and quaternary amino groups; and (5) any combination of any one of (1) to (4); (vii) a coating with ligand-binding proteins and/or their cognate ligands, preferably selected from globulins, particularly immunoglobulins, streptavidin, biotin, Protein A, Protein G; enzymes such as oxidoreductases, transferases, ligases such as polymerases, hydrolases such as proteases, peptidases, nucleases, saccharidases, lipases, lyases, and isomerases; and (viii) a combination of any one of (i) to (vii).

Suitable materials for said magnets include the following elements and their alloys: neodymium-iron, neodymium-iron-boron (e.g. Nd₂Fe₁₄B), cobalt, gadolinium, terbium, dysprosium, iron, nickel, iron oxides, manganese-bismuth, manganese-antimony, manganese-arsenic, yttrium-iron oxides, chromium oxides, europium oxides, and samarium-cobalt. Particularly preferred materials are neodymium-iron and samarium-cobalt.

In a less preferred embodiment, what is referred to as "magnet" may also be implemented using paramagnetic material, in particular in case the magnetic susceptibility of such paramagnetic material is high.

Suitable coatings in accordance with (c)(i) include polypropylene, polyethylene, polystyrene, parylene, titanium nitride, polyimide, chloropolymers, and fluoropolymers, preferably polytetrafluoroethylene (PTFE).

In the methods and use of the invention at least one magnetic body performs a fluctuating or oscillating motion, wherein said motion is triggered by a fluctuating or oscillating magnetic field, wherein preferably said magnetic field is generated by an electric current and/or an electromagnet.

In accordance with the invention, said magnetic body collides with said molecule.

In a further preferred embodiment, at least one non-magnetic particle is present, and said motion of said magnetic body triggers collision of said at least one non-magnetic particle with said molecule.

The latter two embodiments may be practiced as alternatives. As regards the second of the two embodiments, it is of note, though, that generally not only said at least one non-magnetic particle collides with said molecule, but also said magnetic body. In other words, the magnetic body acts as a trigger of collisions of said at least one non-magnetic particle with said molecules, but may in addition also perform direct collisions with said molecule. All these effects generally contribute to fragmenting. The term "collision" refers to a scenario of transient and sufficient spatial proximity of said magnetic body or said non-magnetic particle on the one hand and said molecule on the other hand. Under such circumstances, energy, including kinetic energy, is transferred to said molecule. The transferred energy in turn triggers or contributes to fragmentation of said molecule.

Said non-magnetic beads are distinct from the particles optionally comprised in the magnetic body, and furthermore distinct from paramagnetic beads as commonly used in analytics. The non-magnetic beads may be ceramic beads, polymer beads, glass beads, or metal beads, the metal being a non-magnetic metal. In terms of size, they are preferably in the range between 1 µm and 5 mm such as between 0.1 mm and 2 mm. Also preferred is that said non-magnetic particles have the same or a similar size range as compared to the size of the magnetic body or magnet.

As regards motion, the magnetic body moves up and down and back and forth, wherein the motion may have regular or repeating components but does not have to, and wherein spatial directions are not particularly limited. Also, the magnetic body may rotate about one or more axes, usually in addition to translational motion. For a more precise description of the envisaged types of motion, see further below. To the extent a reactor, i.e. a container, enclosure or vessel is used, the magnetic body may, but does not have to, hit or repeatedly hit a wall of said reactor. Accordingly, the magnetic body, while being in motion all the time (if specific implementations of the method of the invention do not specify otherwise, e.g., an intermittent motion), such motion is preferably not a directed motion. Also, said motion, despite being possibly irregular, generally is about an average position which is located within the mentioned enclosure or container - the magnetic body does not leave the reactor. The motion of the magnetic body generally has one or more translational components; and said average position may be somewhere in the middle of said reactor. As such, the motion is different from the motion performed by a magnetic stirrer - which is a rotation, and the average position of the magnet is at or close to the bottom of the vessel containing the liquid to be mixed or stirred.

The term "oscillating" designates a regular motion, whereas the term "fluctuating" is broader and embraces also irregular motion. There is no particular preference in that respect. In practice, given that often the magnet not only collides with the molecules to be cleaved, but also with at least one wall of a reactor or enclosure used to contain the magnetic body and the molecule, irregular motion occurs more often. In either case, the motion ensures that the magnetic body is brought into contact with said molecule, generally repeatedly, and also repeatedly with all or substantially all molecules to be cleaved to the extent a plurality of molecule or a macroscopic amount thereof is to be processed by the method of the invention. As mentioned above, the motion of said magnetic body might also cause collisions of said molecule with further magnetic bodies and/or non-magnetic particles, to the extent they are present.

As noted above, and in accordance with the invention, the motion of the magnetic body is triggered by a fluctuating or oscillating magnetic field.

A magnetic field is a common means of controlling position and/or motion of a magnet. Given that in accordance with the invention, the magnetic body moves, use is made of a fluctuating or oscillating magnetic field in the invention. Any such magnetic field may be useful.

Preference is given to said magnetic field being generated by an electric current. It is well established that electric and magnetic fields are interrelated; in particular that an electric current generates a magnetic field. As a consequence, controlling the electric current is a means of controlling the magnetic field generated thereby.

Alternatively, or in addition, but less preferred, said magnetic field may be generated or modulated, respectively, by an external magnet. The term "external" means that such magnet is not located within said reactor. The external magnet may be a permanent magnet. When using an external magnet, the magnetic field generated thereby may be rendered fluctuating or oscillating by corresponding movement of said external magnet relative to said at least one magnetic body.

In a preferred embodiment, said magnetic field is generated by an electromagnet. As used herein, the term "electromagnet" embraces, in its simplest implementation, a piece of an electric conductor through which an electric current is flowing when in use. For better control of the magnetic field or for the purpose of generating stronger magnetic fields, particular implementations of the electromagnet are envisaged which are subject of preferred embodiments disclosed further below.

In a certain implementation, said electric current fluctuates or oscillates. This behavior may also be referred to as a generic "wave". The amount of an electric current is known as amperage.

In a certain implementation, amperage of said electric current as a function of time is (i) a rectangular function; (ii) a sinusoidal function; (iii) a triangular function; (iv) a sawtooth function; or (v) a combination or convolution of any one of (i) to (iv).

Given that the electric current oscillates or fluctuates, this also applies to patterns (i) to (v), i.e., said rectangular and said triangular functions are in fact repeating rectangular and triangular functions. The term "pattern" designates a series of events where a given basic event is repeated at least once. In a wider sense, repetition does not have to be a precise repetition - the lengths of e.g. rectangles in a time graph may change (which effectively amounts to a change of frequency, preferred frequencies as well as preferred time dependencies of frequencies being specified further below).

All options (i) to (v) are also referred to as "alternating current" herein.

Particularly preferred is said rectangular function (as referred to as rectangular wave or square wave), more specifically the patterns of repeating rectangular functions. The inventors surprisingly found that this pattern triggers particularly vigorous motion of the magnetic body, wherein such vigorous motion is particularly efficient in terms of cleavage. In said rectangular function, the time intervals of high current and low current (or the current being off) may be the same or different. Means to control the length of said time intervals are known to the skilled person, e.g. those referred to as pulse width modulation (PWM). Of note, the energy transferred to the reaction mixture is not only governed by frequency and amplitude of the electric current, but also by the relative duration of said time intervals.

In a certain implementation of the method of the first aspect, said colliding transfers an amount of energy to said molecule which is sufficient to cleave at least one covalent bond. It is well established that collision is a means of transferring energy from one body to another. The energy received by the receiving body may be converted into internal energy of said body (here a molecule) and trigger its fragmentation.

The above is a means to specify the overall result of a specific implementation chosen: The energy transferred to the molecule to be cleaved shall be such that at least one covalent bond in said molecule receives the amount of energy required for dissociation.

As an estimate, the amount of energy transferred by the magnetic body to a covalent bond is equal to or less than the energy transferred by the magnetic field to the magnetic body. The energy in a magnetic field per unit volume is defined by *E_{mag} = ½ B*²/*µ₀;* for definitions of *B* and *µ₀* see further below. *E_{mag}* in turn is equal or less than the energy of the electric current which causes the magnetic field. The latter energy can be estimated as *E_{curr} = U I t, U* being the voltage and *I* the amperage of the current generating the magnetic field, and *t* is the time during which the current flows. In other words, controlling any one of *B, U, I* and *t* is a means of controlling the amount of energy transferred by the magnetic body to a covalent bond.

Having said that, there are other means of specifying the detailed implementation of the method of the first aspect. This includes specifying one or more of a plurality of parameters which can be more directly controlled or measured. These parameters include frequency and amperage of the current, and may furthermore include dimensions of a reactor and a coil, to the extent use is made thereof. Also, the strength of the magnetic field, preferably at the site of the magnetic body, is a means of quantitatively specifying implementation details. In all these cases, preference is given to those parameter values or combinations of parameter values which ensure that the above requirement (transfer of sufficient energy to cleave a bond) is met. In the following, preferred ranges of the mentioned parameters are specified.

In a certain implementation, said electric current fluctuates or oscillates with a given frequency, preferably a frequency of 0.1 Hz to 20 MHz, more preferably 10 Hz to 2 kHz, yet more preferably 50 to 500 Hz or 90 to 300 Hz or 100 to 200 Hz. It is understood that these measures apply not only to sinusoidal current, but to all current profiles specified herein, including, e.g., the repeated rectangular pattern. The term frequency may also apply to fluctuations, i.e., time-dependent behavior which is not regular (such regular time behavior also referred to as "oscillation" herein), and is a means to characterize the timescale of fluctuations. In such a case, the term "frequency" is understood as referring to the average frequency of the fluctuation.

It turns out that at least for reactors with a volume in the one-digit to two-digit mL range as well as for the wells of a standard 96-well plate, lower frequencies between 80 and 300 Hz work particularly well, whereas significantly higher frequencies such as around 1000 Hz, while inducing vibration of the magnetic body, might not trigger the full range of motion which covers a significant portion of the volume of the reactor. This does not mean that higher frequencies are not beneficial. Also, they may be used in conjunction with lower frequencies (see below).

More generally speaking, a preferred frequency range is a range which ensures that the magnetic body not only vibrates or rotates but performs a translational motion which explores the entire volume or substantially the entire volume of the material to be processed with the method of the invention. In those implementations which make use of a reactor and the material to be processed is in solution or suspension, said volume is the total volume of said solution or suspension as contained in said reactor. In other words, while guidance is given above with regard to reactors with a volume in the one-digit to two-digit mL range and 96-well plates, the frequency ranges may need adaptation for reactors with significantly smaller volume, significantly larger volume, or special geometries. To give an example, it is expected that for smaller volumes such as the wells of high-density microtiter plates (e.g. 1536-well plates) higher frequencies, e.g. of about 1 kHz such as above 200 Hz, lead to a motion of the magnetic body which is comparable to the motion seen in larger vessels at lower frequencies. In any case, a skilled person provided with the guidance given in this specification, can explore and optimize in a straightforward manner the parameters controlling motion of said at least one magnetic body. As explained further above preference is given to the magnetic body performing translational motion, preferably in addition to rotation.

In a certain implementation, said frequency is kept constant throughout while said method is performed.

In another implementation, said frequency changes as a function of time.

In a further implementation, more than one frequency is applied at a given point in time. In such a case, each frequency of such a plurality of frequencies may be chosen from any of the preferred intervals given above. Particularly preferred in case of two frequencies is that the first frequency is between 50 Hz and 500 Hz and the second frequency between 80 Hz and 20 MHz. In other words, this preferred embodiment provides for the superposition of a plurality of frequencies.

More than one frequency includes 2, 3, 4, 5, 6, 7, 8, 9 and 10 different frequencies. Such plurality of frequencies may be applied throughout in place of a single frequency - which means that they are applied during the entire performance of the method. Also, a plurality of frequencies, or different pluralities of frequencies may be applied in different time intervals within a longer time span. Within said longer time span, and in addition to time intervals where more than one frequency is applied, there may be one or more, such as 2, 3, 4, 5, 6, 7, 8, 9, or 10 time intervals where only one frequency is applied.

The inventors surprisingly found that a regime where more than one frequency is applied performs superior in terms of yield. "Yield" designates the relative quantity of fragmented molecules compared to the total number of (unfragmented) molecules at the beginning of the method.

In a further implementation, said frequency is, and, in case more than one frequency is applied, the frequencies are not constant over time, and preferably is/are switched or gradually changed between two or more frequencies, preferably in a periodic manner.

Exemplary regimes are (120 Hz - 1000 Hz)ₙ, (200 Hz - 1000 Hz)ₙ, or (100 Hz - 800 Hz)ₙ, wherein n is an integer, e.g. between 2 and 1000, such as between 10 and 100, and specifies the number of times the frequency pattern in brackets is to be repeated.

The duration of the time interval with constant frequency and /or constant amperage is not particularly limited. Envisaged are time intervals between 1 sec and 1 day, such as between 1 min and 1 hr.

In a further implementation, said electric current (a) has an amperage I between 20 mA and 100 A, preferably between 0,1 and 20 A; (b) exposes said magnet to a magnetic field strength between 0,02 and 10⁹ A/m, preferably between 10 and 10⁶ A/m; and/or (c) is applied for a time span t between 1 sec and 1 week, such as between 10 min and 5 hrs.

Keeping in mind that, as disclosed above, the amperage as a function of time fluctuates on a timescale governed by the frequencies disclosed herein, there is no constant amperage on the timescale of the fluctuation. Yet, for practical purposes, and in line with established practice in electrodynamics, an alternating current may be quantified in terms of its average amperage. The above values are average amperages in that sense. Of note, the average is preferably over the time scale of the fluctuations. That means, to the extent intermittent current is used, there will be an average amperage, preferably within the ranges specified above, when the current is on, and there will be zero amperage when the current is off.

The magnetic field strength *H* determines the intensity of the field and is measured in A per meter. *H* has to be distinguished from the magnetic flux density B which is particularly relevant in setting where a core is used to re-enforce the magnetic field of an electric current; see below.

In a certain implementation, the amplitude of fluctuation or oscillation is (a) constant; or (b) changes over time, preferably on a timescale which is slower than the timescale of said fluctuation or oscillation.

This embodiment refers to the amplitude of motion of said electric current. The amplitude of oscillation or fluctuation of an electric current is governed by the amperage.

In a further implementation, said current is intermittent and/or said amperage changes over time, preferably in a periodic manner. This change over time is generally on a time scale which is slower than the time scale defined by the frequency of the alternating current. In other words, if an alternating current changes over time in the sense of this embodiment, the time dependency of the current is a superposition of two patterns or waves: a generally fast fluctuation which is inherent to an alternating current, and a generally slower change.

An exemplary intermittent pattern is a repetition of the sequence on (1 min) - off (1 min). Other preferred time intervals are given above. Advantages of intermittent patterns allow for keeping temperature constant or substantially constant, especially if it is observed that the contents of the reactor is heating up.

In a further implementation, a power of between 0 and 1000 W, preferably between 1 and 200 W is applied.

In a further implementation, the electric current is powered by an electric power source. Preferably, the electric power source has an electric potential or voltage *U* in the range between about 0 and 240 V such as between 0.1 and 75 V. These values refer to the mean voltage applied.

In a further implementation, said electromagnet comprises at least one coil, wherein preferably said coil (a) has a plurality of or windings, such as between 1 and 10⁴, preferably between 10 and 1000; and/or (b) comprises at least one Helmholtz coil; and/or (c) comprises at least one core.

Exemplary numbers of a plurality of coils are 2, 3, 4, 5, 6, 7, 8, 9, 10, 20, 50, 100, 150, 200, 300, 400 and 500.

The term "Helmholtz coil" is established in the art and refers to an arrangement of typically two identical coils spaced apart such that their axes of rotational symmetry align or coincide. The magnetic field in the space between the coils is particularly homogenous and/or particularly strong. Arrangements of at least one Helmholtz coil may be two Helmholtz coils.

Further arrangements of a plurality of coils are known to provide for extended spatial regions of particularly homogeneous magnetic field. A further example is a Maxwell coil.

A core serves to re-enforce the effect of the magnetic field. A core is preferably made of ferromagnetic material such as iron, in particular soft iron. The magnetic flux density *B* is related to magnetic field strength as follows: *B = µᵣµ₀ H. µ₀* is the magnetic permeability of vacuum and as such a fundamental physical constant. *µᵣ* on the other hand is the relative permeability and determines the degree of re-enforcement of the magnetic flux density by a given material, e.g. a ferromagnetic core when under the influence of a magnetic field. Typically, *µ*ᵣ for ferromagnetic materials to be used as a core is between 10³ and 10⁶ such as between 200000 and 400000, e.g. about 300000.

Suitable core materials comprise powdered metals, laminated metals, annealed metals such as annealed iron, ceramics, and solid metals.

Preferred values of *B* in the absence of a core are between 10⁻⁸ and 10⁴ Tesla (T), such as between 10⁻⁵ and 1 T. If a core with a specific relative permeability is used, the values of B are to be multiplied with said relative permeability. As such, preferred values of B in the presence of a core are between 10⁻² and 10⁹ such as between 1 and 10⁶ T. Generally, these values refer to B at the site of the magnetic body or within said reactor.

In terms of geometry, preferred coils are circular. Preferred diameters are between 1 mm and 1 m or 1 mm and 0,5 m, such as between 2 mm and 300 mm or 2 mm and 200 mm. Envisaged are also different geometries such as coils with a square, a rectangular or a triangular shape (i.e., all or part of the windings are a square, a rectangle or a triangle). Finally, and noting that a coil is not an indispensable requirement for an electromagnet, also an arrangement of two antiparallel wires may be used ("antiparallel" referring to the direction of the electric current flowing through the two wires at a given point in time).

In a further implementation, more than one coil is used, preferably between 2 and 10⁴ such 2, 3, 4, 5, 6, 7, 8, 9, or 10 coils, or between 10 and 1000 coils. It is understood that each coil may have one or a plurality of windings, preferred numbers of windings being disclosed herein above.

In a further implementation, the obtained fragments are amenable to mass spectrometry. This is consistent with what is stated above, namely that fragmenting is a means of obtaining moieties the size of which is particularly suitable for analysis by mass spectrometry. What size range is most suitable depends on the application at issue. The above disclosed parameter which control the motion of said magnetic body are means of controlling the size of the obtained fragments.

In a further implementation, said sample is of biological origin and preferably (i) is or comprises a solution or suspension of said molecule, e.g. said sample comprises said molecule in purified form, a mixture of proteins, polypeptides and/or peptides, or is or comprises a bodily fluid such as blood, serum, plasma, cerebrospinal fluid, sputum or urine; (ii) is or comprises a cell such as a prokaryotic or eukaryotic cell, e.g. said sample is or comprises a suspension of cells; (iii) is or comprises a virus, e.g. said sample is a suspension of viruses; and/or (iv) is or comprises a tissue, e.g. muscle tissue or brain tissue.

In a certain implementation, said sample is from a healthy organism, a diseased organism or an organism which has been exposed to a stimulus, chemical or drug. Comparing entire proteomes across a plurality of states (healthy vs. diseased; prior to vs. after exposure to a stimulus) is of particular interest in the field of diagnostics and benefits from the present invention. Stimuli may include temperature or a change thereof, the concentration of a gas or a change thereof, and exposure to UV radiation.

In a further implementation, said sample comprises a cell, and/or a tissue, and said cell or said tissue is lysed by said motion. The present inventors recognized earlier that cellular material can be lysed under the influence of a moving magnet; see WO 2020/002577 which is incorporated by reference in its entirety.

In a certain implementation, said sample comprises a virus, and said virus is inactivated or disintegrated by said motion.

Having regard to viruses, and noting that the method provides for both disintegrating the viruses as well as fragmenting their proteins, the invention also provides a convenient method of epitope mapping. As known in the art, an epitope is a moiety recognized by an antibody. The epitope may be a stretch of amino acids which are contiguous in the primary sequence, but does not have to be. In case of three-dimensional epitopes, sequences which are separated from each other in the primary sequence may, upon folding, end up in spatial proximity and thereby define an epitope.

The term "antibody" is not particularly limited. Preferred antibodies include naturally occurring immunoglobulins of all families such as IgG, IgM, IgE, preferably of mammalian or human origin, but also camelid antibodies and antigen-binding molecules having structures which are not naturally occurring (fragments of antibodies, single-chain antibodies, labelled antibodies, conjugates of antibodies such as antibody-drug conjugates etc.).

An exemplary method of epitope mapping of the invention comprises the method of the first aspect applied to a virus, followed by adding one or more antibodies of interest to the obtained mixture of fragments, allowing the binding of fragments to said one or more antibodies, and identifying the bound fragments, preferably by mass spectrometry.

Preferably, antibodies are coupled to beads. This facilitates their handling. Antibodies may originate from individuals who developed immunity against a certain viral pathogen.

Also preferred is that after said allowing of binding, the obtained antibody-fragment complexes are washed. This removes fragments which do not bind to the antibody.

Prior to identifying, it is preferred to elute the bound fragments. This breaks up the antibody-fragment complexes and allows for downstream processing of the binding fragments.

By knowing all binding fragments, the epitope is identified or at least the region where the epitope is located becomes apparent.

As such, the present disclosure provides a method of epitope mapping, said method comprising fragmenting a protein comprising said epitope using at least one moving magnetic body, adding at least one antibody of interest, separating fragments which bind to said antibody from non-binding fragments, and identifying said fragments which bind said antibody.

Preferably, said protein is a viral protein, more preferably a capsid protein.

Of note, the viral proteins do not have to be provided in the context of viral particles. This means that epitope mapping may also be performed using viral proteins or already disintegrated viruses as starting material.

In an alternative, not the antibody, but the fragments obtained by the method of the invention may be immobilized on a carrier or bound to a matrix or particle. Such carrier, matrix or particles may be used to capture antibodies capable of binding to specific fragments. In a yet further alternative, immobilization may be dispensed with altogether.

The notion of interaction mapping is not confined to antibody/antigen interactions. Indeed, the method may be employed to characterize protein/protein interactions in general.

As such, the present invention provides a method of identifying a site on a first protein which is capable or suspected to be capable of binding to a second protein or a binding partner, said method comprising fragmenting said first protein using at least one moving magnetic body, adding said second protein or said binding partner, separating fragments which bind to said second protein or said binding partner from non-binding fragments, and identifying said fragments which bind said second protein or binding partner, thereby identifying said site, wherein said at least one magnetic body performs a fluctuating or oscillating motion, wherein said motion is triggered by a fluctuating or oscillating magnetic field, wherein said magnetic body collides with said molecule, and wherein under such circumstances energy is transferred to said molecule triggering or contributing to fragmenting said molecule.

The term "binding partner" is more general than the recited "second protein". Indeed, this method is not limited to protein-protein interactions, but may be extended or instead be applied to protein-nucleic acid interactions and protein-small molecule interactions.

In a further implementation, said magnetic field triggers at least two motions of said magnetic body, said motions differing in their kinetic energy, wherein said at least two motions occur simultaneously or during different time intervals.

This embodiment provides for a fine-tuning of what the magnetic body does to the sample.

One example is concomitant lysis of cells and fragmentation of the proteins, polypeptides and peptides comprised in said cells. Depending on the type of cell to be lysed, and depending on the desired size range of the fragments, different settings of the device controlling the magnetic body's motion (for details of such device which is also subject of this invention, see further below) at different times during practicing the method of the invention may be desirable. Generally, when performing lysis and protein fragmentation, nucleic acids will fragment concomitantly.

A further example is the abovementioned disintegration of viruses, concomitant with fragmentation of the proteins contained in the viral particle. Here, a change in the setting of said device, e.g. once the virus is disintegrated may be beneficial in order to optimize yield of protein fragmentation and/or to ensure that fragments of the desired size are obtained.

In the context of the abovementioned epitope mapping, a rather broad range of fragments sizes is of interest. A broad range in this context is, e.g. from about 5 to about 100 amino acids. Such broad range, including longer and unique fragments as well as very short ones, is particularly apt for epitope identification.

In a further preferred embodiment, said method of the first aspect further comprises the step of (b) exposing said sample to heat such as boiling; denaturing said sample, e.g. by ultrasonication; adding detergent to said sample; and/or adding a chaotropic agent to said sample, wherein preferably step (b) is performed prior to or concomitantly with step (a), i.e., fragmenting in accordance with the first aspect. Such additional measures may be chosen depending on the specific application under consideration. They may improve the yield of lysis (to the extent cellular material is comprised in the sample) and/or the yield of fragmentation.

Preferred detergents are sodium dodecyl sulfate, sodium deoxycholate, Triton X-100, Tween 20 and CHAPS.

Preferred chaotropic agents are guanidinium hydrochloride, urea and thiourea.

In a further preferred embodiment, said method of the first aspect further comprises the step of (c) chemically modifying said molecule and/or the fragments obtained from said molecule.

In a particularly preferred embodiment, said chemically modifying is selected from (ca) reducing a disulfide; (cb) alkylating a thiol group such as a cysteine residue; (cc) cross-linking; and (cd) any combination of (ca), (cb) and (cc), the combination of (ca) and (cb) being preferred.

These are measures which are performed routinely in the course of sample preparation for mass spectrometry. In accordance with the present invention, they may be performed concomitantly with fragmenting. Of note, in accordance with the present invention, reduction and alkylation are not compulsory. This applies particularly to the above mentioned middle-down approach where larger fragments are of interest.

As such, it is particularly preferred that steps (a), (ca), and (cb) are performed at the same time.

In other words, in an aspect related to the latter embodiment, the present disclosure provides a method of preparing a sample for analysis in a mass spectrometer, said sample comprising at least one protein, polypeptide or peptide molecule, said method comprising (1) adding a reducing agent and an alkylating agent to said sample; and (2) fragmenting said molecule using at least one moving magnetic body. It is understood that step (2) is performed in the presence of said agents added in step (1).

For example, steps (1) and (2) may be performed at the same time, or step (2) may directly follow step (1).

In a further preferred implementation, an inert viscous liquid; a gel such as a polyacrylamide gel or agarose gel; an aerogel and/or a zeolith is/are added to said sample. Without wishing to be bound by a specific theory, it is considered that by embedding the molecule(s) to be fragmented in a gel or a viscous liquid, is a means of enhancing energy transfer from the magnetic body or, to the extent present, any non-magnetic particle, to said molecule(s), which in turn enhances yield.

When using a polyacrylamide gel, this generally involves running a polyacrylamide gel electrophoresis of a sample comprising said at least one molecule. The region of interest may then be cut out from the gel or the gel as a whole may be processed in accordance with the first aspect, i.e., subjected to fragmenting. Electrophoresis is not a requirement, though. One can also add the components needed for formation of a polyacrylamide gel to the sample to be processed, let the gel form, and subject it then to the method of the first aspect.

When agarose is to be used, agarose may be added to the sample to be processed, boiled, and allowed to cool. This yields and agarose gel with the molecules to be fragmented being embedded in the gel. Alternatively, agarose may be boiled prior to combining it with the sample, thereby avoiding exposure of the sample to boiling temperature.

In a further implementation, a protease such as trypsin, LysC, GluC, AspN, ArgC or chymotrypsin is added.

As noted above, one of the advantages of the present invention is that chemical and enzymatic means of hydrolysis or cleavage are rendered dispensable in that they may be replaced by "magnetic fragmentation". Yet, in certain cases it may be desirable to modulate the spectrum of fragments obtained by said magnetic fragmentation by the addition of - as such art-established - proteases.

Moreover, also chemicals facilitating cleavage of proteins may be added. For example, CNBr has been described to cleave at Met residues. Formic acid has a preference for Asp-Pro bonds in terms of hydrolysis, and hydroxylamine for Asn-Gly bonds. 2-nitro-5-thiocyano benzoic acid cleaves at Cys residues. Yet further, iron and copper salts facilitate the generation of reactive oxygen species which in turn react with proteins to degrade them.

In another implementation, one or more of the following are added to the sample: a surfactant; a detergent; a buffer; an acid; a base; a chaotropic agent; a kosmotropic agent; a salt; and a solvent, preferably an organic solvent. Moreover, further agents commonly used in sample preparation such as phosphorylase inhibitors may be added as well.

Particularly preferred acids are acetic acid, formic acid, and trifluoroacetic acid.

Particularly preferred organic solvents are acetonitrile, ethanol, methanol, isopropanol, and trifluoro ethanol.

In a further preferred embodiment, the method of the invention further comprises the step of (d) cleaning up and/or enriching the obtained fragments, preferably by means of filtering, non-covalent binding and/or covalent binding, wherein non-covalent binding is preferably to reversed-phase material, forward-phase material, ion exchange material, affinity-binding material, material with chelating properties or paramagnetic particles, and covalent binding is preferably with a reagent capable of forming a conjugate with a functional group such as an amine group of any of said fragments.

In a further implementation, said method further comprises the step of allowing said sample to react with a probe or ligand, wherein preferably said probe is a DNA probe and/or an RNA probe.

In a further preferred embodiment, said method further comprises the step of (e) labeling said molecule and/or the fragments obtained from said molecule, wherein preferably labeling of said fragments is effected after said cleaning and/or enriching of said fragments, wherein said labeling is done by reacting a functional group of said molecule or of a fragment thereof with a reagent capable of forming a conjugate with said functional group, wherein said reagent capable of forming a conjugate is preferably a tag which is detectable by mass spectrometry. The label used is not particularly limited. Given that mass spectrometry is the preferred analytical method in accordance with this invention, preference is given to labels which are detectable by this method, and furthermore to labels which can be provided in differently isotope labelled forms. Different isotope labelling is a convenient way to have labels which are distinguishable in the mass spectrum but behave identical or similar in terms of chemistry, including the chemistry of coupling the label to an analyte (here protein, polypeptide or peptide, including the fragments delivered by the method of the first aspect).

In a certain implementation, said functional group is selected from primary amine group, a carboxy group and a thiol.

Especially preferred is that said functional group is a primary amine group and said tag is an activated ester such as an N-hydroxy succinimide (NHS) ester and/or is provided in at least two differently isotope-labeled forms. Preferably, said differently isotope-labeled forms are such that the overall nominal mass is the same for all forms (which are also referred to as a set of "isobaric tags").

Preferably, said tag is amenable to fragmentation in the mass spectrometer, preferably by gas phase fragmentation, e.g. collision induced dissociation (CID), electron transfer dissociation or UV fragmentation. Of note, such fragmentation is to be held distinct from the fragmentation of proteins, polypeptides and peptides in accordance with the invention - the former refers to the tag and occurs in the spectrometer, and the latter occurs prior to spectrometry and affects the mentioned proteinaceous molecules.

This type of labeling is generally performed at the fragment level, i.e., proteins and polypeptides (and possibly longer peptides) are fragmented with the method of the first aspect, and thereafter the obtained fragments are labelled. Preference is given to stable isotopes.

Yet, this is not the only possibility of labeling proteinaceous molecules for the purpose of analysis in a mass spectrometer. For example, metabolic labeling (one implementation being termed "SILAC") may be performed. This provides for the incorporation of isotope-labeled building blocks into a protein, e.g. while it is being synthesized on the ribosome. In such a case, labeling occurs prior to fragmentation, and samples, in particular differently labeled samples, may be pooled prior to fragmentation.

More than one sample may be processed with the methods of the invention, wherein it is preferred that different samples are labeled differently. Different samples may be healthy and diseased samples, or a sample taken prior to and after administration of an agent or drug or exposure to a stimulus, for details see above.

Differently labeled samples may be pooled. This is means of enhancing throughput on a mass spectrometer. Different labeling ensures that the different samples comprised in a pooled sample are distinguishable in the mass spectrum. In other words, pooling will generally happen after labeling. Also, it is preferred to perform pooling after fragmentation, in particular in those instances where the labeling procedure is applied to fragments obtained by the method of the invention.

In a more general sense, pooling embraces the addition of standards. This permits protein quantitation or renders said quantitation more accurate.

In a further implementation, the average fragment length deviates from the fragment length obtained by digestion of said molecule with a protease, wherein preferably the average fragment length obtained with said method is larger. This is a distinct advantage of the invention. It arises from "magnetic fragmenting" being mechanistically distinct from the art-established enzymatic fragmenting, and furthermore from the fact that the motion of the magnetic body can be conveniently controlled and fine-tuned by a multitude of parameters which are disclosed above. As explained above in an exemplary manner, magnetic fragmenting may be used to obtained average fragments sizes which are larger than tryptic fragments, e.g. in the range from 30 to 100 amino acids.

It is furthermore preferred that the probability density of fragment size is broader when using the method of the present invention as compared to enzymatic or chemical fragmentation. In this implementation, a broad range of fragment size is obtained which may embrace both conventional fragment size as obtained by, e.g., a tryptic digestion as well as larger fragments in the sense of the above discussed middle-down approach.

In a second aspect, the present invention provides an analytic method comprising the method of any one of the preceding claims, and a step of performing mass spectrometry of the obtained fragments.

The method of the second aspect, in a preferred embodiment, includes a step of chromatographic separation of the fragments prior to mass spectrometry, generally by liquid chromatography (LC). Preferably, this is implemented as HPLC. Also preferred is that the chromatographic material is reverse phase material such as C18 material.

In a certain implementation of the method of the second aspect, said method furthermore comprises a computer-implemented step of determining sequence and/or identity of said molecule. This step is effected by analyzing the fragments, e.g. by determining their sequence from the mass spectrum. Once the sequences of a sufficient number of fragments is known, sequence assembly algorithms may be employed to infer the original sequence of the protein or polypeptide which gave rise to the fragments.

In a third aspect, the invention provides a use of a magnetic body and means for generating a fluctuating or oscillating magnetic field for fragmenting a protein, polypeptide or peptide molecule, wherein a fluctuating or oscillating motion of the magnetic body is generated by said magnetic field, wherein said magnetic body collides with said molecule, and wherein under such circumstances energy is transferred to said molecule triggering or contributing to fragmenting said molecule.

Preferred embodiments of the above methods define preferred embodiments of said use of the invention.

The present disclosure furthermore relates to a kit comprising or consisting of (i) at least one magnetic body; and (ii) a vessel or an array of vessels each configured to receive said magnetic body, and a sample comprising at least one protein, polypeptide or peptide molecule.

In a certain implementation, said kit further comprises or further consists of (iii) a reducing agent; and (iv) an alkylating agent.

Preferred reducing agents in accordance with all aspects are dithiothreitol and tris(2-carboxyethyl) phosphine.

Preferred alkylating agents in accordance with all aspects include haloacetic acids, haloacetamides, haloalkane amides and N,N-dialkyl haloalkane amides. Preferably, each occurrence of alkane is independently chosen from linear or branched C1 to C5 alkane such as methyl, ethyl and iso-propyl. Halogen (or "halo") include chloro, bromo and iodo. Alkyl moieties may be substituted, a preferred substituent being hydroxy.

These agents may be used for the reducing step and the alkylating step of the method of the invention as disclosed above.

In a further implementation, said kit, preferably in addition to said alkylating agent and said reducing agent, further comprises or further consists of (v) one, more or all of a surfactant, a chaotropic agent, a denaturing agent, and an organic solvent; (vi) at least one buffer; (vii) non-magnetic particles; and/or (vii) a manual comprising instructions for performing a method of the invention.

Preferred surfactants, preferred chaotropic agents and preferred solvents are disclosed herein above. These agents may have denaturing properties.

Buffers serve to establish and maintain the pH value of the sample during the various stages of the method of the invention. An exemplary pH value is between 8 and 8.5. Suitable buffer substances for handling biological molecules such as proteins, polypeptides, peptides and nucleic acids are known to the skilled person and available from a multitude of manufacturers.

Said non-magnetic particles are those which are disclosed further above. Said particles, also referred to as "beads" herein, may be metal beads or ceramic beads.

In certain implementations of said kit, the kit is tailored for the above disclosed methods of epitope mapping. Accordingly, said kit further comprises or further consists of Protein A and/or Protein G, and optionally one or more buffers suitable to perform said method of epitope mapping. Preferably, Protein A and/or Protein G would be immobilized on a surface or carrier such as beads or a plate. Alternatively, said kit further comprises or further consists of a surface or carrier with reactive moieties. Such reactive moieties may be NHS, epoxy or carboxy groups. They serve to immobilize fragments obtained in step (a) of the methods of the invention to said surface or carrier. Again, beads and plates are suitable implementations of said surface or carrier.

The words "further consists of" as herein refer to those implementations where the kit consists of a closed number of constituents. In the case above, these are items (i) to (iii) as defined above and item (iv) which is subject of said implementation.

The above disclosed vessel is not particularly limited.

Useful vessels include those which are generally used in the field of molecular biology and in vitro diagnostics. Such vessels are generally free or substantially free of contaminants, chemically inert, and/or have surfaces with low binding capacity.

Important in the context of the present invention is that vessels have at least one wall which does not shield magnetic fields. Preferably, the entire vessel is made of a material which does not shield magnetic fields. Suitable materials include plastic, polymers such as polypropylene, glass, and ceramics. Keeping the requirement of magnetic permeability in mind, also metals may be used.

Exemplary and preferred vessels are those which are configured to hold a volume of 5 µL to 1 L, preferably between 10 µL to 50 mL, more preferably configured to hold volumes of any of 30 µL, 40 µL, 100 µL, 150 µL, 200 µL, 250 µL, 500 µL, 1 mL, 1.5 mL, 2 mL, 5 mL, 15 mL and 50 mL.

Vessels may be arranged in arrays, such as the common formats (96, 384 or 1546 wells).

In an implementation, a vessel may be equipped with a filter layer or a frit, such vessel also being referred to as cartridge. This allows for convenient separation of fragments (optionally labelled fragments) from any other material. The solution comprising the fragments may be passed through the filter or frit, e.g. by exerting positive pressure on the cartridge, connecting it to vacuum, or centrifuging. The whole process may be performed by a liquid handling machine. The filtrate may be captured in a second vessel or a dedicated compartment within the same vessel.

In a certain implementation, said filter as comprised in said vessel is a molecular weight cut-off filter. Such filters are known in the art and retain all material which has a molecular weight higher than the cut-off value while letting pass through all molecules with a molecular weight below the cut-off.

An accordingly equipped cartridge provides for a preferred embodiment of the method of the invention. In particular, said method, when performed in said cartridge (said magnetic body being located above said filter, i.e., where high molecular weight material is retained), provides for continuous removal of those fragments which are below the cut-off. By passing liquid through said cartridge while inducing motion of the magnet, a further fragmentation of those fragments which are capable of passing the filter will not occur.

This setup has distinct advantages. It may be used to obtain fragments with a narrow size distribution. Also, it may be operated in continuous, i.e., flow-through mode.

In a further implementation, said kit further comprises or further consists of a labeling agent and/or a cross-linking agent.

The present disclosure furthermore relates to a device comprising or consisting of an electric conductor, preferably at least one coil, more preferably a Helmholtz coil; and a vessel or an array of vessels, wherein the opening of said coil is configured to accommodate said vessel or said array of vessels.

Said vessel and said array of vessels are those defined above in relation to the kit.

The term "accommodate" means that said coil has an opening wide enough such that said vessel or said array fits inside said opening, the consequence being that the contents of said vessel(s) is located where the magnetic field generated by said coil when in use is particularly strong and/or particularly homogeneous. Preferably, and in case of said vessel having a circular cross section (such as in case of a cylindrical vessel), the inner diameter of said coil (a circular coil in that case) is only slightly wider than the outer diameter of said vessel. "Slightly wider" may mean between 0.01 and 10% such as between 0.1 and 1% wider.

Similarly, the coil in case of an array of vessels (such as a microtiter plate) may be such that it is just slightly wider than said array. If said array is rectangular in shape, a coil such as a Helmholtz coil with a rectangular shape may be employed.

In a preferred embodiment, said device further comprises or further consists of (iii) at least one magnetic body, preferably at least one magnetic body per vessel.

Such design provides for said electric conductor or said coil to be configured such that said at least one magnetic body is under the influence of a magnetic field generated by said electric conductor or coil when in use.

It goes without saying that preferred embodiments of said magnetic body are those disclosed herein, e.g. in conjunction with the method of the first aspect. Generally speaking, preferred embodiments of one aspect define, *mutatis mutandis,* preferred embodiments of another aspect.

In a certain implementation, said device further comprises or further consists of (iv) a control unit configured to cause said at least one magnetic body to perform a fluctuating or oscillating motion when is use. More specifically, this control unit is configured to deliver any of the preferred time profiles of electric current as described in detail in relation to the method of this invention. The control unit may further comprise a power source or an adapter to be connected to an electric plug.

The present disclosure furthermore relates to a computer-implemented method of analyzing a mass spectrum obtained from a sample which has been prepared by the method of the first or second aspect, said computer-implemented method comprising the step of assembling the sequences of the obtained fragments to obtain the sequence of the protein or polypeptide they originate from.

Preferably, such assembling is done without resorting to sequence information known beforehand. To explain further, a common approach in the analysis of mass spectra is the use of databases of fragments such as tryptic fragments of known proteins. The present invention, by providing fragments of a diverse range of sizes, permits a complete and unambiguous mapping of the sequence of an entire protein and thereby renders such databases dispensable. The approach in accordance with the invention may also be termed *de novo* sequencing by mass spectrometry.

The Figures show:
- **Figure 1:**: Number of peptide or fragment identifications (ID). Yeast and Liver samples were either process by a regular sample preparation (Reg. / Standard) or by magnetic fragmentation (Mag. / Magnetic System). "Tryptic" peptides contain a K- or R- amino acid at their C-terminus while "Fragment" peptides can end with any other amino acid.
- **Figure 2:**: Sequence coverage as observed with different implementation of the invention.
- **Figure 3:**: Exemplary distribution of fragment sizes as obtained with the method described in Example 3.

The Examples illustrate the invention.

### Example 1

### Improvement of lysis and digestion when applying the method of the invention in addition to proteolytic digestion

### Materials

Fresh *Saccharomyces cerevisiae* and Mouse liver samples were used at a quantity corresponding to 100 µg protein content. Buffers and enzymes from the iST kit (P.O.00001, PreOmics GmbH) were used throughout this experiment.

### Methods

### Standard iST sample preparation:

Sample preparation was carried out according to the PreOmics standard protocol for yeast samples and according to the mammalian tissue protocol for liver samples. For cell lysis and protein denaturation, yeast pellets (approx. 100 µg protein content) were resuspended in 50 µl lyse buffer, boiled at 95 °C and 1000 rpm for 10 min and sheared in a Diagenode Bioruptor (10 cycles, 30 sec on, 30 sec off). Liver samples (1-2 mg wet weight with approx. 100 µg protein content) were resuspended in 100 µl lyse buffer, sheared in the Diagenode Bioruptor (10 cycles, 30 sec on, 30 sec off) with glass beads to facilitate tissue lysis and boiled at 95 °C and 1000 rpm for 10 min. All samples were further processed according to the manufacturer instructions. After elution, purified peptides were dried in a vacuum centrifuge and resuspended in LC-Load. Samples were analyzed on a ThermoFisher Scientific Easy n-LC 1000 system coupled with a Thermo LTQ Orbitrap XL. Peptides were separated on a home-made C₁₈ column applying a 45 min gradient and tandem mass spectrometry was performed using a DDA Top 10 method. The MS/MS data was searched against a yeast database using the MaxQuant software with default settings, except that the unspecific digestion mode was selected.

### iST sample preparation with cell lysis and digestion on novel Magnetic System:

Yeast samples containing 100 µg of protein were resuspended in 50 µl lyse buffer and either first boiled at 95 °C and 1000 rpm for 10 min or directly mixed with 50 µl trypsin/LysC solution. For cell lysis and protein digestion, samples were incubated on the magnetic system with a 3 mm round Neodymium magnet at a magnetic flux density of approx. 1 mT and 120 Hz for 60 min. Next, 100 µl stop buffer were added and peptides were purified and analyzed as described in Standard iST samples preparation.

### Results

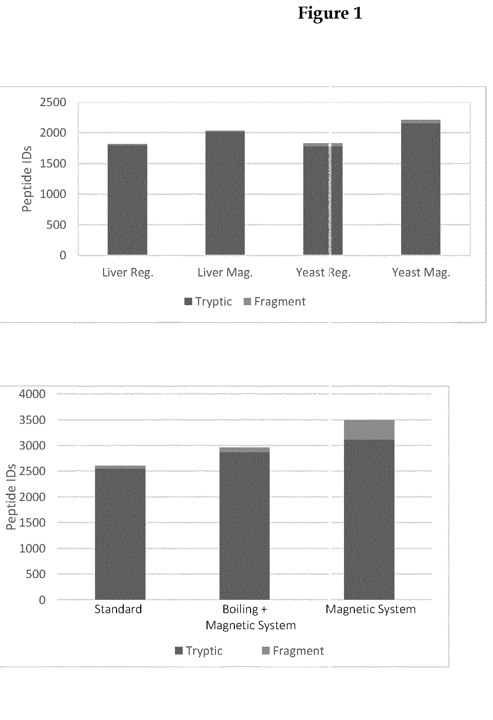

See Figure 1.

### Discussion

The magnetic system clearly improves the overall peptide identifications for yeast cells as well as tissue samples. When comparing the process in conjunction to additional boiling, the process appears to be free standing and additional boiling is not required to achieve best results. The system can directly be employed and can replace traditional lysis plus mixing during digestion. Best results were achieved when using the magnetic system for lysis and digestion in a directly combined manner.

### Example 2

### Fragmentation of an individual protein by the method of the invention (in absence of any protease)

### Materials

50 µg Carbonic anhydrase (bovine erythrocytes; C7025-1VL) at 10 mg/ml in ddH2O was used for the following experiments. For protein extraction and fragmentation, a magnetic system prototype with a Helmholtz coil of 50 windings was developed and run with cylindric 3 mm x 2 mm Samarium Cobalt magnets (MagnetExpert, F412SC-250). 1.2mm steel beads or 1.4mm ceramic beads were added where indicated. For peptide clean-up, the iST kit was used (PreOmics GmbH, P.O.00001).

### Methods

Samples were incubated on the magnetic system prototype with a cylindric samarium cobalt magnet and steel beads or ceramic beads where indicated. The system was used at a magnetic flux density of approx. 1 mT and 120 Hz for 60 minutes. Fragment peptides were directly analyzed on a ThermoFisher Scientific Easy n-LC 1200 system coupled to a LTQ Orbitrap XL. Peptides were purified according to the iST manual and separated on a home-made C₁₈ column applying a 45 min gradient and tandem mass spectrometry was performed using a DDA Top 10 method. The MS/MS data was searched against a carbonic anhydrase database using the MaxQuant software with default settings, except that the unspecific digestion mode was selected.

### Results

Peptides of various lengths were generated. Fragments with differences of exactly one amino acid length are commonly observed. This provides a near perfect coverage of every possible peptide fragment option in the given analytical range. For example, fragments of sequence ANGERQSP, ANGERQSPV, ANGERQSPVD, ANGERQSPVDI, ANGERQSPVDID, ANGERQSPVDIDT, ANGERQSPVDIDTK, etc. were measured as individual peptides.

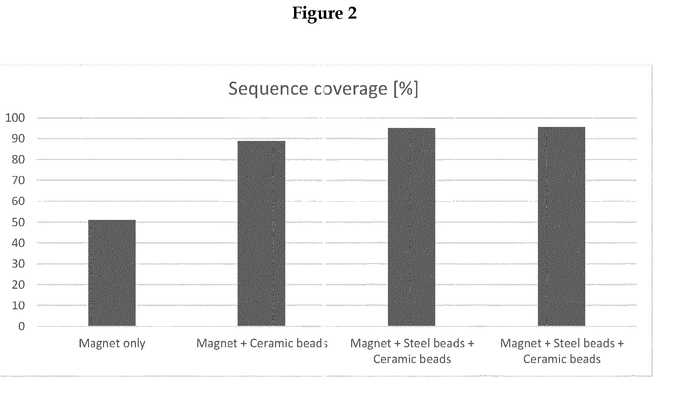

See Figure 2.

### Discussion

The magnetic system can be used to fragment a protein in nearly every peptide composition possible. With only 1.5h sample preparation from start to measurement, the carbonic anhydrase could be fragmented and generated a peptide based protein sequence coverage of >95%. Four Experiments combined generated a complete sequence coverage. The peptide fragments obtained with the method of the invention can be used to sequence a protein, since fragments of every length combination are generated, differing only in the mass of a single amino acid.

By using these differences, the original sequence can furthermore be deduced.

### Example 3

### Protein fragmentation on a proteome-wide scale

### Materials

Fresh *Saccharomyces cerevisiae* cell pellets containing approximately 100 µg were used for the following experiments. For protein extraction and fragmentation, a magnetic system prototype with a coil of 250 windings and inner diameter of 12mm was developed and run with 2 mm round Neodymium magnets. Peptide purification and preparation for LC-MS measurement was performed with iST cartridges and buffers from PreOmics GmbH (iST Kit, P.O.00001). Water was commercially purchased from Fisher Scientific (W6-212).

### Methods

Yeast pellets containing 100 µg yeast proteins were resuspended in 100 µl ddH20 with pH of 1,4,7, or 10 in 90 µl ddH20 with pH of 1,4,7, or 10 and 10 µl acetonitrile. For protein extraction and fragmentation, samples were incubated on the magnetic system prototype with a 2 mm round Neodymium magnet applying a magnetic flux density of approx. 1.5 mT and 120 Hz for 60 minutes. Next, 100 µl iST stop buffer were added and peptides were purified according to the manufacturer protocol. Peptides were dried in the vacuum centrifuge and resuspended in LC-Load to a final concentration of 2.5 µg/µl. Samples were analyzed on a ThermoFisher Scientific Easy n-LC 1200 system coupled to a LTQ Orbitrap XL. Peptide loads of 5 µg were separated on a home-made C₁₈ column applying a 45 min gradient and tandem mass spectrometry was performed using a DDA Top 10 method. The MS/MS data was searched against a yeast database using the MaxQuant software with default settings, except that the unspecific digestion mode was selected.

### Results

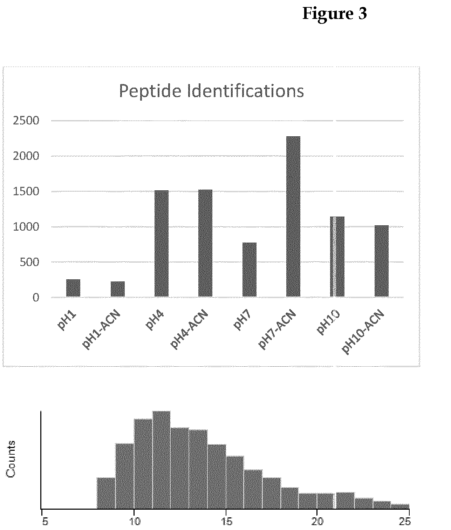

See Figure 3.

### Discussion

The magnetic system as used in this experiment generated peptides of a mean length of 13.2 amino acids which is longer then standard tryptic digestion (approx. 12 amino acid length). The system generates suitable peptide fragments at various pH ranges, also in the presence of organic solvent.

## Claims

1. A method of preparing a sample for an analytic procedure, said sample comprising at least one protein, polypeptide or peptide molecule, and said method comprising
(a) fragmenting said molecule using at least one moving magnetic body, wherein said at least one magnetic body performs a fluctuating or oscillating motion, wherein said motion is triggered by a fluctuating or oscillating magnetic field, wherein said magnetic body collides with said molecule, and wherein under such circumstances energy is transferred to said molecule triggering or contributing to fragmenting said molecule.

2. The method of claim 1, wherein said magnetic field is generated by an electric current and/or an electromagnet.

3. The method of claim 1 or 2, wherein
(i) said fragmenting is a non-enzymatic and non-chemical process; or
(ii) a chemical selected from CNBr, formic acid, hydroxylamine, and 2-nitro-5-thiocyano benzoic acid and/or a protease is added.

4. The method of any one of claims 1 to 3, wherein at least one non-magnetic particle is present, wherein said motion of said magnetic body triggers collision of said at least one non-magnetic particle with said molecule.

5. The method of any one of the preceding claims, wherein said sample is of biological origin and preferably
(i) is or comprises a solution or suspension of said molecule, e.g. said sample comprises said molecule in purified form, a mixture of proteins, polypeptides and/or peptides, or is or comprises a bodily fluid such as blood, serum, plasma, cerebrospinal fluid, sputum or urine;
(ii) is or comprises a cell such as a prokaryotic or eukaryotic cell, e.g. said sample is or comprises a suspension of cells;
(iii) is or comprises a virus, e.g. said sample is a suspension of viruses; and/or
(iv) is or comprises a tissue, e.g. muscle tissue or brain tissue.

6. The method of any one of the preceding claims, further comprising the step of
(b) exposing said sample to heat such as boiling, denaturing said sample, adding detergent to said sample, and/or adding a chaotropic agent to said sample, wherein preferably step (b) is performed prior to or concomitantly with step (a).

7. The method of any of the preceding claims, further comprising the step of
(c) chemically modifying said molecule and/or the fragments obtained from said molecule.

8. The method of any of the preceding claims, wherein said analytic procedure is mass spectrometry (MS).

9. The method of claim 7, wherein said chemically modifying is selected from
(ca) reducing a disulfide;
(cb) alkylating a thiol group such as a cysteine residue;
(cc) cross-linking; and/or
(cd) any combination of (ca), (cb) and (cc), the combination of (ca) and (cb) being preferred;
wherein preferably steps (a), (ca), and (cb) are performed at the same time.

10. The method of any one of the preceding claims, wherein an inert viscous liquid; a gel such as a polyacrylamide gel or agarose gel; an aerogel and/or a zeolith is/are added to said sample.

11. The method of any one of the preceding claims, further comprising the step of
(d) cleaning up and/or enriching the obtained fragments,
preferably by means of filtering, non-covalent binding and/or covalent binding, wherein non-covalent binding is preferably to reversed-phase material, forward-phase material, ion exchange material, affinity-binding material, material with chelating properties or paramagnetic particles, and covalent binding is preferably with a reagent capable of forming a conjugate with an amine group of any of said fragments.

12. The method of any one of the preceding claims, further comprising the step of
(e) labeling said molecule and/or the fragments obtained from said molecule, wherein preferably labeling of said fragments is effected after said cleaning and/or enriching of said fragments, wherein said labeling is done by reacting a functional group of said molecule with a reagent capable of forming a conjugate with said functional group, wherein said reagent capable of forming a conjugate is preferably a tag which is detectable by mass spectrometry.

13. The method of any one of the preceding claims, wherein the size of the magnetic body is between 0.1 mm and 10 cm.

14. The method according to claim 2, wherein said electric current exposes said magnet to a magnetic field strength between 0,02 and 10⁹ A/m.

15. An analytic method comprising the method of any one of the preceding claims, and a step of performing mass spectrometry of the obtained fragments.

16. A method of identifying a site on a first protein which is capable or suspected to be capable of binding to a second protein or a binding partner, said method comprising fragmenting said first protein using at least one moving magnetic body, adding said second protein or said binding partner, separating fragments which bind to said second protein or said binding partner from non-binding fragments, and identifying said fragments which bind said second protein or binding partner, thereby identifying said site,
wherein said at least one magnetic body performs a fluctuating or oscillating motion, wherein said motion is triggered by a fluctuating or oscillating magnetic field, wherein said magnetic body collides with said molecule, and wherein under such circumstances energy is transferred to said molecule triggering or contributing to fragmenting said molecule,
and wherein preferably said first protein is an antigen and said second protein is an antibody.

17. Use of a magnetic body and means for generating a fluctuating or oscillating magnetic field for fragmenting a protein, polypeptide or peptide molecule, wherein a fluctuating or oscillating motion of the magnetic body is generated by said magnetic field, wherein said magnetic body collides with said molecule, and wherein under such circumstances energy is transferred to said molecule triggering or contributing to fragmenting said molecule.

## Patentansprüche

1. Verfahren zur Vorbereitung einer Probe für einen Analysevorgang, wobei die Probe mindestens ein Protein-, Polypeptid- oder Peptidmolekül umfasst und wobei das Verfahren Folgendes umfasst:
(a) Fragmentieren des Moleküls unter Verwendung mindestens eines sich bewegenden Magnetkörpers, wobei der mindestens eine Magnetkörper eine fluktuierende oder oszillierende Bewegung ausführt, wobei die Bewegung durch ein fluktuierendes oder oszillierendes Magnetfeld ausgelöst wird, wobei der Magnetkörper mit dem Molekül kollidiert und wobei unter solchen Umständen Energie auf das Molekül übertragen wird, wodurch das Fragmentieren des Moleküls ausgelöst oder dazu beigetragen wird.

2. Verfahren nach Anspruch 1, wobei das Magnetfeld durch einen elektrischen Strom und/oder einen Elektromagneten erzeugt wird.

3. Verfahren nach Anspruch 1 oder 2, wobei
(i) die Fragmentierung ein nichtenzymatischer und nichtchemischer Prozess ist; oder
(ii) eine Chemikalie, ausgewählt aus CNBr, Ameisensäure, Hydroxylamin und 2-Nitro-5-thiocyanobenzoesäure und/oder eine Protease zugesetzt wird.

4. Verfahren nach einem der Ansprüche 1 bis 3, wobei mindestens ein nichtmagnetisches Partikel vorhanden ist, wobei die Bewegung des Magnetkörpers eine Kollision des mindestens einen nichtmagnetischen Partikels mit dem Molekül auslöst.

5. Verfahren nach einem der vorhergehenden Ansprüche, wobei die Probe biologischen Ursprungs ist und vorzugsweise
(i) eine Lösung oder Suspension des Moleküls ist oder umfasst, wobei die Probe z. B. das Molekül in gereinigter Form, eine Mischung von Proteinen, Polypeptiden und/oder Peptiden umfasst oder eine Körperflüssigkeit wie z. B. Blut, Serum, Plasma, Hirn-Rückenmark-Flüssigkeit, Sputum oder Urin ist oder umfasst;
(ii) eine Zelle wie z. B. eine prokaryotische oder eukaryotische Zelle ist oder umfasst, wobei die Probe z. B. eine Suspension von Zellen ist oder umfasst;
(iii) ein Virus ist oder umfasst, wobei die Probe z. B. eine Suspension von Viren ist; und/oder
(iv) ein Gewebe, z. B. Muskelgewebe oder Hirngewebe, ist oder umfasst.

6. Verfahren nach einem der vorhergehenden Ansprüche, das ferner den Schritt umfasst,
(b) die Probe Hitze, z. B. durch Kochen, auszusetzen, die Probe zu denaturieren, der Probe ein Detergens zuzusetzen und/oder der Probe ein chaotropes Mittel zuzusetzen, wobei Schritt (b) vorzugsweise vor oder gleichzeitig mit Schritt (a) durchgeführt wird.

7. Verfahren nach einem der vorhergehenden Ansprüche, das ferner den Schritt umfasst,
(c) das Molekül und/oder die aus dem Molekül erhaltenen Fragmente chemisch zu modifizieren.

8. Verfahren nach einem der vorhergehenden Ansprüche, wobei der Analysevorgang Massenspektrometrie (MS) ist.

9. Verfahren nach Anspruch 7, wobei die chemische Modifizierung aus Folgendem ausgewählt ist:
(ca) Reduzieren eines Disulfids;
(cb) Alkylieren einer Thiolgruppe, wie z. B. eines Cysteinrests;
(cc) Vernetzen; und/oder
(cd) einer beliebigen Kombination aus (ca), (cb) und (cc), wobei die Kombination aus (ca) und (cb) bevorzugt ist;
wobei vorzugsweise die Schritte (a), (ca) und (cb) gleichzeitig durchgeführt werden.

10. Verfahren nach einem der vorhergehenden Ansprüche, wobei der Probe eine inerte viskose Flüssigkeit; ein Gel wie z. B. ein Polyacrylamidgel oder ein Agarosegel; ein Aerogel und/oder ein Zeolith zugesetzt wird/werden.

11. Verfahren nach einem der vorhergehenden Ansprüche, das ferner den Schritt umfasst,
(d) die erhaltenen Fragmente zu reinigen und/oder anzureichern,
vorzugsweise durch Filtrieren, nichtkovalente Bindung und/oder kovalente Bindung, wobei die nichtkovalente Bindung vorzugsweise an Umkehrphasenmaterial, Vorwärtsphasenmaterial, Ionenaustauschmaterial, affinitätsbindendem Material, Material mit chelatbildenden Eigenschaften oder paramagnetischen Partikeln erfolgt und die kovalente Bindung vorzugsweise mit einem Reagenz erfolgt, das in der Lage ist, ein Konjugat mit einer Aminogruppe eines der Fragmente zu bilden.

12. Verfahren nach einem der vorhergehenden Ansprüche, das ferner den Schritt umfasst,
(e) das Moleküls und/oder die aus dem Molekül erhaltenen Fragmente zu markieren, wobei das Markieren der Fragmente vorzugsweise nach dem Reinigen und/oder Anreichern der Fragmente erfolgt, wobei das Markieren durch Reagieren einer funktionellen Gruppe des Moleküls mit einem Reagenz erfolgt, das in der Lage ist, ein Konjugat mit der funktionellen Gruppe zu bilden, wobei das Reagenz, das in der Lage ist, ein Konjugat zu bilden, vorzugsweise ein Etikett ist, das durch Massenspektrometrie nachweisbar ist.

13. Verfahren nach einem der vorhergehenden Ansprüche, wobei die Größe des Magnetkörpers zwischen 0,1 mm und 10 cm liegt.

14. Verfahren nach Anspruch 2, wobei der elektrische Strom den Magneten einer Magnetfeldstärke zwischen 0,02 und 10⁹ A/m aussetzt.

15. Analyseverfahren, umfassend das Verfahren nach einem der vorhergehenden Ansprüche und einen Schritt der Durchführung einer Massenspektrometrie der erhaltenen Fragmente.

16. Verfahren zum Identifizieren einer Stelle auf einem ersten Protein, die in der Lage ist oder vermutlich in der Lage ist, an ein zweites Protein oder einen Bindungspartner zu binden, wobei das Verfahren Folgendes umfasst:
Fragmentieren des ersten Proteins unter Verwendung mindestens eines sich bewegenden Magnetkörpers, Zusetzen des zweiten Proteins oder des Bindungspartners, Trennen von Fragmenten, die an das zweite Protein oder den Bindungspartner binden, von nicht bindenden Fragmenten und Identifizieren der Fragmente, die an das zweite Protein oder den Bindungspartner binden, wodurch die Stelle identifiziert wird,
wobei der mindestens eine Magnetkörper eine fluktuierende oder oszillierende Bewegung ausführt, wobei die Bewegung durch ein fluktuierendes oder oszillierendes Magnetfeld ausgelöst wird, wobei der Magnetkörper mit dem Molekül kollidiert und wobei unter solchen Umständen Energie auf das Molekül übertragen wird, wodurch die Fragmentierung des Moleküls ausgelöst oder dazu beigetragen wird,
und wobei vorzugsweise das erste Protein ein Antigen und das zweite Protein ein Antikörper ist.

17. Verwendung eines Magnetkörpers und eines Mittels zur Erzeugung eines fluktuierenden oder oszillierenden Magnetfelds zum Fragmentieren eines Protein-, Polypeptid- oder Peptidmoleküls, wobei eine fluktuierende oder oszillierende Bewegung des Magnetkörpers durch das Magnetfeld erzeugt wird, wobei der Magnetkörper mit dem Molekül kollidiert und wobei unter solchen Umständen Energie auf das Molekül übertragen wird, wodurch die Fragmentierung des Moleküls ausgelöst oder dazu beigetragen wird.

## Revendications

1. Procédé de préparation d'un échantillon pour une procédure analytique, ledit échantillon comprenant au moins une molécule de protéine, de polypeptide ou de peptide, et ledit procédé comprenant
(a) la fragmentation de ladite molécule en utilisant au moins un corps magnétique mobile, dans lequel ledit au moins un corps magnétique réalise un mouvement fluctuant ou oscillant,
dans lequel ledit mouvement est déclenché par un champ magnétique fluctuant ou oscillant,
dans lequel ledit corps magnétique entre en collision avec ladite molécule, et dans lequel, dans de telles circonstances, de l'énergie est transférée à ladite molécule, déclenchant ou contribuant à la fragmentation de ladite molécule.

2. Procédé selon la revendication 1, dans lequel ledit champ magnétique est généré par un courant électrique et/ou un électroaimant.

3. Procédé selon la revendication 1 ou 2, dans lequel
(i) ladite fragmentation est un processus non enzymatique et non chimique ; ou
(ii) un produit chimique choisi parmi le CNBr, l'acide formique, l'hydroxylamine et l'acide 2-nitro-5-thiocyano benzoïque et/ou une protéase est ajouté.

4. Procédé selon l'une quelconque des revendications 1 à 3, dans lequel au moins une particule amagnétique est présente, dans lequel ledit mouvement dudit corps magnétique déclenche une collision de ladite au moins une particule amagnétique avec ladite molécule.

5. Procédé selon l'une quelconque des revendications précédentes, dans lequel ledit échantillon est d'origine biologique et de préférence
(i) est ou comprend une solution ou une suspension de ladite molécule, par exemple ledit échantillon comprend ladite molécule sous forme purifiée, un mélange de protéines, de polypeptides et/ou de peptides, ou est ou comprend un fluide corporel tel que du sang, du sérum, du plasma, du liquide céphalo-rachidien, des expectorations ou de l'urine ;
(ii) est ou comprend une cellule telle qu'une cellule procaryote ou eucaryote, par exemple ledit échantillon est ou comprend une suspension de cellules ;
(iii) est ou comprend un virus, par exemple ledit échantillon est une suspension de virus ; et/ou
(iv) est ou comprend un tissu, par exemple un tissu musculaire ou un tissu cérébral.

6. Procédé selon l'une quelconque des revendications précédentes, comprenant en outre l'étape de
(b) exposition dudit échantillon à une chaleur telle qu'une ébullition, dénaturation dudit échantillon, ajout d'un détergent audit échantillon, et/ou ajout d'un agent chaotrope audit échantillon, dans lequel de préférence, l'étape (b) est réalisée avant, ou de manière concomitante avec, l'étape (a).

7. Procédé selon l'une quelconque des revendications précédentes, comprenant en outre l'étape de
(c) modification chimique de ladite molécule et/ou des fragments obtenus à partir de ladite molécule.

8. Procédé selon l'une quelconque des revendications précédentes, dans lequel ladite procédure analytique est une spectrométrie de masse (MS).

9. Procédé selon la revendication 7, dans lequel ladite modification chimique est choisie parmi
(ca) une réduction d'un disulfure ;
(cb) une alkylation d'un groupe thiol tel qu'un résidu cystéine ;
(cc) une réticulation ; et/ou
(cd) toute combinaison de (ca), (cb) et (cc), la combinaison de (ca) et(cb) étant préférée ;
dans lequel de préférence les étapes (a), (ca) et (cb) sont réalisées en même temps.

10. Procédé selon l'une quelconque des revendications précédentes, dans lequel un liquide visqueux inerte ; un gel tel qu'un gel de polyacrylamide ou un gel d'agarose ; un aérogel et/ou une zéolite est/sont ajouté(s) audit échantillon.

11. Procédé selon l'une quelconque des revendications précédentes, comprenant en outre l'étape de
(d) nettoyage et/ou enrichissement des fragments obtenus,
de préférence par filtration, liaison non covalente et/ou liaison covalente, dans lequel la liaison non covalente est de préférence à un matériau en phase inversée, un matériau en phase directe, un matériau échangeur d'ions, un matériau à liaison d'affinité, un matériau ayant des propriétés chélatantes ou des particules paramagnétiques, et la liaison covalente est de préférence avec un réactif capable de former un conjugué avec un groupe amine de l'un quelconque desdits fragments.

12. Procédé selon l'une quelconque des revendications précédentes, comprenant en outre l'étape de
(e) marquage de ladite molécule et/ou des fragments obtenus à partir de ladite molécule, dans lequel de préférence le marquage desdits fragments est effectué après ledit nettoyage et/ou enrichissement desdits fragments, dans lequel ledit marquage est fait par réaction d'un groupe fonctionnel de ladite molécule avec un réactif capable de former un conjugué avec ledit groupe fonctionnel, dans lequel ledit réactif capable de former un conjugué est de préférence un marqueur qui est détectable par une spectrométrie de masse.

13. Procédé selon l'une quelconque des revendications précédentes, dans lequel la taille du corps magnétique est entre 0,1 mm et 10 cm.

14. Procédé selon la revendication 2, dans lequel ledit courant électrique expose ledit aimant à une intensité de champ magnétique entre 0,02 et 10⁹ A/m.

15. Procédé analytique comprenant le procédé de l'une quelconque des revendications précédentes, et une étape de réalisation d'une spectrométrie de masse des fragments obtenus.

16. Procédé d'identification d'un site sur une première protéine qui est capable ou suspectée d'être capable de se lier à une seconde protéine ou à un partenaire de liaison, ledit procédé comprenant la fragmentation de ladite première protéine en utilisant au moins un corps magnétique mobile, l'ajout de ladite seconde protéine ou dudit partenaire de liaison, la séparation des fragments qui se lient à ladite seconde protéine ou audit partenaire de liaison d'avec des fragments qui ne se lient pas, et l'identification desdits fragments qui se lient à ladite seconde protéine ou audit partenaire de liaison, identifiant ainsi ledit site,
dans lequel ledit au moins un corps magnétique réalise un mouvement fluctuant ou oscillant, dans lequel ledit mouvement est déclenché par un champ magnétique fluctuant ou oscillant, dans lequel ledit corps magnétique entre en collision avec ladite molécule, et dans lequel, dans de telles circonstances, de l'énergie est transférée à ladite molécule, déclenchant ou contribuant à la fragmentation de ladite molécule,
et dans lequel de préférence, ladite première protéine est un antigène et ladite seconde protéine est un anticorps.

17. Utilisation d'un corps magnétique et de moyens pour générer un champ magnétique fluctuant ou oscillant pour fragmenter une molécule de protéine, de polypeptide ou de peptide,
dans laquelle un mouvement fluctuant ou oscillant du corps magnétique est généré par ledit champ magnétique, dans laquelle ledit corps magnétique entre en collision avec ladite molécule, et
dans laquelle, dans de telles circonstances, de l'énergie est transférée à ladite molécule, déclenchant ou contribuant à la fragmentation de ladite molécule.
